# EUROPEAN PATENT APPLICATION

(11) **EP 4 549 469 A1**
(43) Date of publication of application: **07.05.2025**
(21) Application number: 23206698.5
(22) Date of filing: 30.10.2023
(51) Int. Cl.: C07K 16/30, C07K 16/40, A61K 39/395, A61P 35/00, A61P 35/04

(54) **ANTIBODY TARGETING MELANOMA CELLS**

(71) Applicant: Eberhard Karls Universität Tübingen Medizinische Fakultät, 72074 Tübingen (DE)
(72) Inventor: Flatz, Lukas, 3074 Muri bei Bern (CH); Purde, Mette-Triin, 9007 St. Gallen (CH); Walter, Vincent, 72070 Tübingen (DE)
(74) Representative: Witte, Weller & Partner Patentanwälte mbB

(57) **Abstract**

The present invention relates to an antibody or fragment thereof specifically binding to tyrosinase-related protein 2 (TRP2), an antibody or fragment thereof for use in the prophylaxis and/or treatment of cancer, preferably melanoma, a pharmaceutical composition comprising said antibody or fragment thereof, a nucleic acid encoding said antibody or fragment thereof, a vector comprising said nucleic acid, a prokaryotic or eukaryotic host cell comprising said vector, and to a method for the prophylaxis and/or treatment of cancer, preferably melanoma, in a living being, comprising the administration of a prophylactically and/or therapeutically effective amount of said antibody or fragment thereof or said pharmaceutical composition.

## Description

The present invention relates to an antibody or fragment thereof specifically binding to tyrosinase-related protein 2 (TRP2), an antibody or fragment thereof for use in the prophylaxis and/or treatment of cancer, preferably melanoma, a pharmaceutical composition comprising said antibody or fragment thereof, a nucleic acid encoding said antibody or fragment thereof, a vector comprising said nucleic acid, a prokaryotic or eukaryotic host cell comprising said vector, and to a method for the prophylaxis and/or treatment of cancer, preferably melanoma, in a living being, comprising the administration of a prophylactically and/or therapeutically effective amount of said antibody or fragment thereof or said pharmaceutical composition.

### BACKGROUND

Melanoma, also redundantly known as malignant melanoma, is a type of cancer that develops from the pigment-producing cells known as melanocytes. Melanomas typically occur in the skin, but may rarely occur in the mouth, intestines, or eye. The primary cause of melanoma is ultraviolet light (UV) exposure in those with low levels of the skin pigment melanin. According to GLOBOCAN 2020, malignant melanoma was diagnosed in 324,635 people and caused 57,043 deaths in 2020. Melanoma is the most lethal skin cancer worldwide due to its high metastasis potential. Patients with metastatic melanoma have reduced survival compared with patients in earlier disease stages. Thus, post-surgery adjuvant therapy is recommended for patients with a high risk of recurrence.

Chemotherapy drugs such as dacarbazine have been the backbone of metastatic melanoma treatment since FDA approval in 1975; however, its efficacy in terms of survival has never been proven in a randomized controlled trial.

Small-molecule targeted therapies work by blocking the genes involved in pathways for tumor proliferation and survival. The main treatments are BRAF, C-Kit and NRAS inhibitors. These inhibitors work to inhibit the downstream pathways involved in cell proliferation and tumor development due to specific gene mutations. However, melanoma tumors can develop resistance during therapy which can make therapy no longer effective.

Immunotherapy is aimed at stimulating the person's immune system against the tumor, by enhancing the body's own ability to recognize and kill cancer cells. The current approach to treating melanoma with immunotherapy includes three broad categories of treatments including cytokines, immune check point inhibitors, and adoptive cell transfer. However, these treatments are often costly and are associated with considerable toxicity.

An overview of the current options for adjuvant therapy for melanoma can be found in Lao et al. (2022), Current state of adjuvant therapy for melanoma: less is more, or more is better? Am. Soc. Clin. Oncol. Educ Book 42, 1-7.

### SUMMARY

Against this background, it is the object of the present invention to provide an alternative or new agent or compound with which the disadvantages of the prior art can be avoided or at least reduced. In particular, such an agent or compound is to be provided with which cancer, in particular melanoma, can be prevented or treated in a targeted manner, preferably in the context of an adjuvant therapy.

The problem underlying the invention is solved by the provision of an antibody or fragment thereof specifically binding to tyrosinase-related protein 2 (TRP2), characterized in comprising
- as heavy chain variable domain a CDR3 region, said CDR3 region has an amino acid sequence being at least 90% identical to one selected from those in SEQ ID NO: 1, 3, 5, 7, 9, 11, 13, or 15, and/or
- as light chain variable domain a CDR3 region, said CDR3 region has an amino acid sequence being at least 90% identical to one selected from those in SEQ ID NO: 2, 4, 6, 8, 10, 12, 14, or 16.

The inventors were able to identify intra-tumoral antibodies specifically targeting TRP2 in a well-established melanoma mouse model. TRP2-specific antibody clones were identified using single-cell B-cell receptor sequencing and recombinantly produced as monoclonal antibodies. The inventors found that eight antibody clones protect wild-type mice against tumor challenge and were well tolerated in an adjuvant-like setting. Antibodies or fragments thereof carrying the CDR3 heavy and/or light chain region essential for antigen recognition are thus particularly suitable for prophylactic or therapeutic treatment of cancer patients, in particular melanoma patients.

The term "antibody" is intended to include any polypeptide chain-containing molecular structure with a specific shape that fits to and recognizes an epitope, where one or more non-covalent binding interactions may stabilize the complex between the molecular structure and the epitope. The term includes both polyclonal and monoclonal antibodies. The archetypal antibody molecule is the immunoglobulin, and all types of immunoglobulins, IgG, IgM, IgA, IgE, IgD, etc., from all sources, e.g., human, rodent, rabbit, cow, sheep, pig, dog, camelid, other mammals, chicken, other avians, etc., are considered to be "antibodies". A preferred source for producing antibodies useful as starting material according to the invention is rabbits.

According to the invention, "CDR" refers to the complementary determining regions which, along with the framework regions (FR), are parts of the variable regions of the immunoglobulin and T cell receptor chains. CDRs and FRs are defined according to the International ImMunoGeneTics (IMGT) information system; see https://www.imgt.org/.

In an embodiment of the invention the antibody is a humanized antibody, i.e., an immunoglobulin or antibody that includes at least one humanized immunoglobulin or antibody chain (i.e., at least one humanized light or heavy chain). The term "humanized immunoglobulin chain" or "humanized antibody chain" (i.e., a "humanized immunoglobulin light chain" or "humanized immunoglobulin heavy chain") refers to an immunoglobulin or antibody chain (i.e., a light or heavy chain, respectively) having a variable region that includes a variable framework region substantially from a human immunoglobulin or antibody and complementarity determining regions (CDRs) (e.g., at least one CDR, preferably two CDRs, more preferably three CDRs) substantially from a non-human immunoglobulin or antibody, and further includes constant regions (e.g., at least one constant region or portion thereof, in the case of a light chain, and preferably three constant regions in the case of a heavy chain). The term "humanized variable region" (e.g., "humanized light chain variable region" or "humanized heavy chain variable region") refers to a variable region that includes a variable framework region substantially from a human immunoglobulin or antibody and complementarity determining regions (CDRs) substantially from a non-human immunoglobulin or antibody.

"Antibody fragments" comprise a portion of a full length antibody, preferably the variable domain thereof, or at least the antigen binding site thereof, such as any of CDR3, CDR2 or CDR1. Examples of antibody fragments include diabodies, single-chain antibody molecules (scFv or scFab), and multispecific antibodies (e.g. bispecific) formed from antibody fragments.

The antibody or fragment thereof according to the invention can be produced by recombinant means. Methods for recombinant production are widely known in the state of the art and comprise protein expression in prokaryotic and eukaryotic cells with subsequent isolation of the antibody or fragment and usually purification to a pharmaceutically acceptable purity. For the expression of the antibodies or fragments as aforementioned in a host cell, nucleic acids encoding the respective light and/or heavy chains or fragments are inserted into expression vectors by standard methods. Expression is performed in appropriate prokaryotic or eukaryotic host cells like CHO cells, NSO cells, SP2/0 cells, HEK293 cells, COS cells, PER.C6 cells, yeast, or *E.coli* cells, and the antibody or fragment is recovered from the cells (supernatant or cells after lysis). General methods for recombinant production of antibodies are well-known in the state of the art and described, for example, in the review articles of Makrides, S.C. (1999), Protein Expr. Purif. 17, 183-202; Geisse et al. (1996), Protein Expr. Purif. 8, 271-282; Kaufman, R.J. (2000), Mol. Biotechnol. 16, 151-161; Werner, R.G. (1998), J. Drug Res. 48, 870-880.

As used herein, the term "binding" or "specifically binding" refers to the binding of the antibody or fragment thereof to an epitope of the antigen (TRP2), e.g., with purified wild-type TRP2 antigen in an *in vitro* assay such as a plasmon resonance assay (BIAcore, GE-Healthcare Uppsala, Sweden). Preferably, a specifically binding antibody or fragment does not exhibit significant cross-reactivity. An antibody or fragment thereof that "does not exhibit significant cross-reactivity" is one that will not appreciably bind to an undesirable entity, e.g., an undesirable proteinaceous entity. For example, an antibody or fragment that specifically binds to TRP2 will appreciably bind TRP2 but will not significantly react with non-TRP2 proteins or peptides, e.g., non-TRP2 proteins or peptides located in tumors. An antibody specific for a preferred epitope will, for example, not significantly cross-react with remote epitopes on the same protein or peptide. Specific binding can be determined according to any art-recognized means for determining such binding. Preferably, specific binding is determined according to Scatchard analysis and/or competitive binding assays. The affinity of the binding is defined by the terms ka (rate constant for the association of the antibody from the antibody/antigen complex), kD (dissociation constant), and KD (kD/ka). Binding or specifically binding means a binding affinity (KD) of 10⁻⁸ mol/l or less, preferably 10⁻⁹ M to 10⁻¹³ mol/l.

In an embodiment of the invention the antibody or fragment thereof is isolated. The term "isolated" means that the material is removed from its original environment (e.g., the natural environment, if it is naturally occurring). For example, a naturally-occurring antibody or fragment thereof present in a living animal is not isolated, but the same antibody or fragment thereof, separated from some or all of the coexisting materials in the natural system, is isolated.

The inventors have recognized that, in one embodiment of the invention, the antibodies or fragments thereof need not necessarily be sequence identical with the indicated sequences, e.g., of the CDRs or CDR3 respectively. Specific and affine binding of the antibody or fragment is also possible if the binding regions are at least 90% identical with the indicated specific sequences.

"Percent identity" or "percent identical" in turn, when referring to a sequence, means that a sequence is compared to a claimed or described sequence after alignment of the sequence to be compared (the "Compared Sequence") with the described or claimed sequence (the "Reference Sequence"). The percent identity is then determined according to the following formula: percent identity = 100 [1 -(C/R)]
wherein C is the number of differences between the Reference Sequence and the Compared Sequence over the length of alignment between the Reference Sequence and the Compared Sequence, wherein
(i) each amino acid or base in the Reference Sequence that does not have a corresponding aligned base or amino acid in the Compared Sequence and
(ii) each gap in the Reference Sequence and
(iii) each aligned amino acid or base in the Reference Sequence that is different from an aligned base or amino acid in the Compared Sequence, constitutes a difference and
(iv) the alignment has to start at position 1 of the aligned sequences;
and R is the number of amino acids or bases in the Reference Sequence over the length of the alignment with the Compared Sequence with any gap created in the Reference Sequence also being counted as an amino acid or base.

According to the invention, throughout the description and with respect to all embodiments, "at least 90%" identity includes a sequence identity of 90, 91, 92, 93, 94, 95, 96, 97, 98, 99, and 100%.

The antibody or fragment thereof disclosed in accordance with the present invention may also be in "purified" form. The term "purified" does not require absolute purity; rather, it is intended as a relative definition, and can include preparations that are highly purified or preparations that are only partially purified, as those terms are understood by those of skill in the relevant art. For example, individual clones isolated from cellular material have been conventionally purified to electrophoretic homogeneity. Purification of starting material or natural material to at least one order of magnitude, preferably two or three orders, and more preferably four or five orders of magnitude is expressly contemplated. Furthermore, a claimed antibody or fragment thereof which has a purity of preferably 99.999%, or at least 99.99% or 99.9%; and even desirably 99% by weight or greater is expressly encompassed.

The antibody or fragment thereof according to the invention may be in "enriched form". As used herein, the term "enriched" means that the concentration of the material is at least about 2, 5, 10, 100, or 1000 times its natural concentration (for example), advantageously 0.01%, by weight, preferably at least about 0.1% by weight. Enriched preparations of about 0.5%, 1%, 5%, 10%, and 20% by weight are also contemplated. The sequences, constructs, vectors, cells, and other materials comprising the present invention can advantageously be in enriched or isolated form.

Tyrosinase-related protein-2 (TRP2) belongs to the tyrosinase family of melanosomal enzymes involved in melanin synthesis and is expressed in melanocytes and most melanomas. It was initially called DOPAchrome tautomerase because it catalyzes the rearrangement of DOPAchrome to the carboxylated derivative DHICA. The human variant (Entrez: 1638, UniProt: P40126) is a membrane protein that comprises 498 amino acids encoded by the *DCT* (TYRP2) gene.

The findings of the inventors were surprising. For example, TRP2 has already been described in the prior art as a possible target for melanoma therapy; cf. Steitz et al. (2000), Genetic immunization of mice with human tyrosinase-related protein 2: implications for the immunotherapy of melanoma, Int. J. Cancer 86(1):89-94. However, it has not yet been possible to provide specific antibodies or antibody sequences that are suitable for such therapy.

The disclosed amino acid sequences have been identified by the inventors in such intratumoral antibodies or lymph node located antobodies in the respective particular important CDR3 regions, in each case of the heavy and light chain, which are particularly suitable according to the invention to be applied prophylactically or therapeutically for the treatment of cancer, in particular melanoma.

In another embodiment of the invention said antibody or fragment thereof is further characterized in that
- the heavy chain variable domain comprises a CDR2 region, said CDR2 region has an amino acid sequence being at least 90% identical to one selected from those in SEQ ID NO: 17, 19, 21, 23, 25, 27, 29, or 31, and/or
- the light chain variable domain comprises a CDR2 region, said CDR2 region has an amino acid sequence being at least 90% identical to one selected from those in SEQ ID NO: 18, 20, 22, 24, 26, 28, 30, or 32.

In still another embodiment of the invention said antibody or fragment thereof is further characterized in that
- the heavy chain variable domain comprises a CDR1 region, said CDR1 region has an amino acid sequence being at least 90% identical to one selected from those in SEQ ID NO: 33, 35, 37, 39, 41, 43, 45, or 47, and/or
- the light chain variable domain comprises a CDR1 region, said CDR1 region has an amino acid sequence being at least 90% identical to one selected from those in SEQ ID NO: 34, 36, 38, 40, 42, 44, 46, or 48.

This measure provides the two further CDRs, i.e., CDR2 and CDR1, responsible for direct interaction with and binding to the antigen or epitope, i.e., TRP2. By including these additional CDRs the affinity, specificity and selectivity and thus also prophylactic and therapeutic suitability of the antibody according to the invention and fragment thereof are thus further increased.

Another embodiment of the invention provides the antibody or fragment thereof which comprises
- the heavy chain variable domains
   - CDR3 region having an amino acid sequence being at least 90% identical to said in SEQ ID NO: 1 and/or
   - CDR2 region having an amino acid sequence being at least 90% identical to said in SEQ ID NO: 17 and/or
   - CDR1 region having an amino acid sequence being at least 90% identical to said in SEQ ID NO: 33 and/or
- the light chain variable domains
   - CDR3 region having an amino acid sequence being at least 90% identical to said in SEQ ID NO: 2 and/or
   - CDR2 region having an amino acid sequence being at least 90% identical to said in SEQ ID NO: 18 and/or
   - CDR1 region having an amino acid sequence being at least 90% identical to said in SEQ ID NO: 34.

With this measure, the antigen-binding sites responsible for antigen binding and therapeutic efficacy of the particular affine and specific antibody designated by the inventors as 'clone 2' are made available. Thus, the antibody or fragment thereof according to the invention can be readily produced in a known manner.

Another embodiment of the invention provides the antibody or fragment thereof which comprises
- the heavy chain variable domains
   - CDR3 region having an amino acid sequence being at least 90% identical to said in SEQ ID NO: 3 and/or
   - CDR2 region having an amino acid sequence being at least 90% identical to said in SEQ ID NO: 19 and/or
   - CDR1 region having an amino acid sequence being at least 90% identical to said in SEQ ID NO: 35 and/or
- the light chain variable domains
   - CDR3 region having an amino acid sequence being at least 90% identical to said in SEQ ID NO: 4 and/or
   - CDR2 region having an amino acid sequence being at least 90% identical to said in SEQ ID NO: 20 and/or
   - CDR1 region having an amino acid sequence being at least 90% identical to said in SEQ ID NO: 36.

With this measure, the antigen-binding sites responsible for antigen binding and therapeutic efficacy of the particular affine and specific antibody designated by the inventors as 'clone 6' are made available. Thus, the antibody or fragment thereof according to the invention can be readily produced in a known manner.

In another embodiment of the invention said antibody or fragment thereof comprises
- a heavy chain having an amino acid sequence being at least 90% identical to said in SEQ ID NO:49, and/or
- a light chain having an amino acid sequence being at least 90% identical to said in SEQ ID NO:50.

In this embodiment, the entire amino acid sequences of the hypervariable regions of the heavy (IgH) and light chains (IgK) of the antibody of the invention (clone 2) are provided, i.e., not only the sequences of CDR1, 2, and 3, but also the sequences of the intervening 'framework regions' (FRs) FR1, FR2, FR3, and FR4. The production is thus considerably simplified and the resulting antibody and fragment are characterized by particular suitability according to the invention for the prophylaxis and treatment of cancer and in particular melanoma.

In another embodiment of the invention the antibody or fragment thereof comprises
- a heavy chain having an amino acid sequence being at least 90% identical to said in SEQ ID NO:51, and/or
- a light chain having an amino acid sequence being at least 90% identical to said in SEQ ID NO:52.

In this embodiment, the entire amino acid sequences of the hypervariable regions of the heavy (IgH) and light chains (IgK) of the antibody of the invention (clone 6) are provided, i.e., the sequences of CDR1, 2, and 3, and the sequences of intervening FR1, FR2, FR3, and FR4. The production is thus considerably simplified and the resulting antibody and fragment are characterized by particular suitability according to the invention for the prophylaxis and treatment of cancer and in particular melanoma.

In an embodiment of the invention said antibody or fragment thereof specifically binds to mouse and/or human TRP2.

The human and mouse variants of TRP2 show 83.2% amino acid sequence identity. The inventors were able to show that the antibodies of the invention, in particular clones 2 and 6, as well as fragments thereof, exhibit strong and specific binding to both the mouse variant and the human variant. This is particularly advantageous for prophylactic and therapeutic use of the antibody in humans and mice.

Since no anti-TRP2 antibody is yet available in the prior art which is suitable prophylactically and/or therapeutically for use in medicine, especially in the treatment of cancer and in particular melanoma, a further object of the invention relates precisely to such an antibody or fragment thereof configured for a specific binding to tyrosinase-related protein 2 (TRP2). Preferably, this antibody or fragment thereof is the antibody or fragment thereof according to the invention, which is described in detail above.

The features, characteristics, advantages and embodiments mentioned further above apply *mutatis mutandis* also for this subject-matter.

As used herein, "prophylaxis" describes the totality of all measures taken for this purpose to prevent impairment of health by risk factors, diseases or accidents. The prevention of secondary diseases or maldevelopments by timely treatment of a primary disease is also a form of prophylaxis. In relation to the invention is thought, in particular, to the prevention of the development of cancer, especially melanoma.

Prophylaxis of cancer also includes "prevention of metastasis" or "prevention of secondary tumors", i.e., measures aiming to prevent the transmission of cancerous cells from the primary tumor to one or more sites elsewhere in a patient where then secondary tumors develop. This means that the metastasis of the primary, tumor or cancer is prevented, delayed, or reduced and thus the development of secondary tumors is prevented, delayed, or reduced. Preferably the metastasis, i.e., secondary tumors, of the lung are prevented or reduced, which means that metastatic transmission of cancerous cells from the primary tumor to the lung is prevented or reduced.

According to the invention "treatment", as used in this context, refers to therapeutic measures aimed either at eliminating the cause of the disease (causal therapy) or at eliminating the symptoms (symptomatic therapy). The invention, in particular, refers to the treatment of cancer, especially melanoma. Also included is the use of the antibody or fragment thereof according to the invention for use in adjuvant therapy. Adjuvant therapy generally refers to supplementary or supportive therapeutic measures, but in particular after surgical removal of the tumor, e.g., melanoma.

Still another subject-matter of the invention relates to a pharmaceutical composition characterized in comprising an antibody of fragment thereof according to the invention.

The features, characteristics, advantages and embodiments mentioned in relation with the antibody or fragment thereof apply *mutatis mutandis* also for the pharmaceutical composition.

The pharmaceutical composition may comprise a pharmaceutical carrier. As used herein, "pharmaceutical carrier" includes any and all solvents, dispersion media, coatings, antibacterial and antifungal agents, isotonic and absorption delaying agents, and the like that are physiologically compatible. Preferably, the carrier is suitable for intravenous, intramuscular, subcutaneous, parenteral, spinal or epidermal administration (e.g. by injection or infusion).

A pharmaceutical composition of the present invention can be administered by a variety of methods known in the art. As will be appreciated by the skilled artisan, the route and/or mode of administration will vary depending upon the desired results. To administer a compound of the invention by certain routes of administration, it may be necessary to coat the compound with, or co-administer the compound with, a material to prevent its inactivation. For example, the compound may be administered to a subject in an appropriate carrier, for example, liposomes, or a diluent. Pharmaceutically acceptable diluents include saline and aqueous buffer solutions. Pharmaceutical carriers include sterile aqueous solutions or dispersions and sterile powders for the extemporaneous preparation of sterile injectable solutions or dispersion. The use of such media and agents for pharmaceutically active substances is known in the art.

These pharmaceutical compositions may also contain adjuvants such as preservatives, wetting agents, emulsifying agents and dispersing agents. Prevention of presence of microorganisms may be ensured both by sterilization procedures, *supra,* and by the inclusion of various antibacterial and antifungal agents, for example, paraben, chlorobutanol, phenol, sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

Actual dosage levels of the active ingredient, i.e., antibody or fragment thereof, in the pharmaceutical composition of the present invention may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions of the present invention employed, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

In an embodiment the antibody and/or fragment thereof are the only active ingredients of the pharmaceutical composition. In another embodiment, the pharmaceutical composition may contain additional active agents, such as anti-cancer or anti-melanoma compounds, e.g., traditional chemotherapeutics, interferons, imatinib, anti-PD-1 antibodies, BRAF and/or MEK inhibitors, etc.

A still further subject-matter according to the invention relates to a kit comprising:
a) a container comprising the antibody or fragment thereof according to the invention in solution or in lyophilized formulation;
b) optionally, a second container containing a diluent or reconstituting solution for the lyophilized formulation;
c) optionally, instructions for (i) use of the solution or (ii) reconstitution and/or use of the lyophilized formulation.

The kit may further comprise one or more of (iii) a buffer, (iv) a diluent, (v) a filter, (vi) a needle, or (v) a syringe. The container is preferably a bottle, a vial, a syringe or test tube; and it may be a multi-use container. The pharmaceutical composition is preferably lyophilized.

The kit of the present invention preferably comprises a lyophilized formulation of the present invention in a suitable container and instructions for its reconstitution and/or use. Suitable containers include, for example, bottles, vials (e.g., dual chamber vials), syringes (such as dual chamber syringes) and test tubes. The container may be formed from a variety of materials such as glass or plastic. Preferably the kit and/or container contain/s instructions on or associated with the container that indicates directions for reconstitution and/or use. For example, the label may indicate that the lyophilized formulation is to be reconstituted to peptide concentrations as described above. The label may further indicate that the formulation is useful or intended for subcutaneous administration.

The features, characteristics, advantages and embodiments mentioned in relation with the antibody or fragment thereof apply *mutatis mutandis* also to the kit.

Still another subject-matter of the invention relates to a nucleic acid encoding an antibody or fragment thereof according to the invention.

This measure allows the recombinant production of the antibody or fragment thereof, according to the invention, in an advantageous manner. Methods for recombinant production are widely known in the state of the art as has been described further above.

The features, characteristics, advantages and embodiments mentioned in relation with the antibody or fragment thereof apply *mutatis mutandis* also for the nucleic acid.

The terms "nucleic acid" or "nucleic acid molecule", as used herein interchangeably, are intended to include DNA molecules and RNA molecules. A nucleic acid molecule may be single-stranded or double-stranded, but preferably is double-stranded DNA.

Yet, another subject-matter of the present invention is a vector, preferably an expression vector, comprising the nucleic acid according to the invention, and, optionally, regulatory elements necessary for expression in a prokaryotic and/or eukaryotic cell.

A "vector" is a nucleic acid molecule, in particular self-replicating, which transfers an inserted nucleic acid molecule into and/or between host cells. The term includes vectors that function primarily for insertion of DNA or RNA into a cell (e.g., chromosomal integration), replication of vectors that function primarily for the replication of DNA or RNA, and expression vectors that function for transcription and/or translation of the DNA or RNA. Also included are vectors that provide more than one of the functions as described.

An "expression vector" is a polynucleotide which, when introduced into an appropriate host cell, can be transcribed and translated into a polypeptide. An "expression system" usually refers to a suitable host cell comprised of an expression vector that can function to yield a desired expression product. "Regulatory elements" of expression vectors are well known to the skilled artisan and include, e.g., promotors, enhancers, polyadenylation signals and transcription termination sequence, etc.

The features, characteristics, advantages and embodiments mentioned in relation with the antibody or fragment thereof apply *mutatis mutandis* also for the vector.

Still another subject-matter of the invention is a prokaryotic or eukaryotic host cell comprising the vector according to the invention.

The term "host cell" as used in the current application denotes any kind of cellular system which can be engineered to generate the antibodies according to the current invention. "Host cell" herein typically refers to a cell or non-human organism genetically engineered to produce the anti-TRP2 antibodies or fragments thereof according to the invention. These hosts and host cells include mammalian cells, bacterial, yeast, insect cells, plant cells and transgenic plants or animals such as rodents, plants and bovines. Typically, antibodies or antibody fragments are expressed in mammalian, bacterial and yeast cells. In one embodiment HEK293 cells and CHO cells are used as host cells.

The features, characteristics, advantages and embodiments mentioned in relation with the antibody or fragment thereof apply *mutatis mutandis* also for the prokaryotic or eukaryotic host cell.

Another subject-matter of the invention relates to a method for the prophylaxis and/or treatment of cancer, preferably melanoma, in a living being, comprising the administration of a prophylactically and/or therapeutically effective amount of the antibody or fragment thereof and/or the pharmaceutical composition according to the invention.

The features, characteristics, advantages and embodiments mentioned in relation with the antibody or fragment thereof apply *mutatis mutandis* also for the method according to the invention.

A "living being" refer to any subject or organism, including mammals, in particular humans and mice.

The invention is now further explained by means of embodiments and examples resulting in additional features, characteristics and advantages of the invention. The embodiments and examples are of pure illustrative nature and do not limit the scope or range of the invention. The features mentioned in the specific embodiments and examples are features of the invention and may be seen as general features which are not applicable in the specific embodiment or example but also in an isolated manner in the context of any embodiment or example of the invention.

The invention is now further described and explained in more detail by referring to the following non-limiting examples and figures.

### BRIEF DESCRIPTION OF THE FIGURES

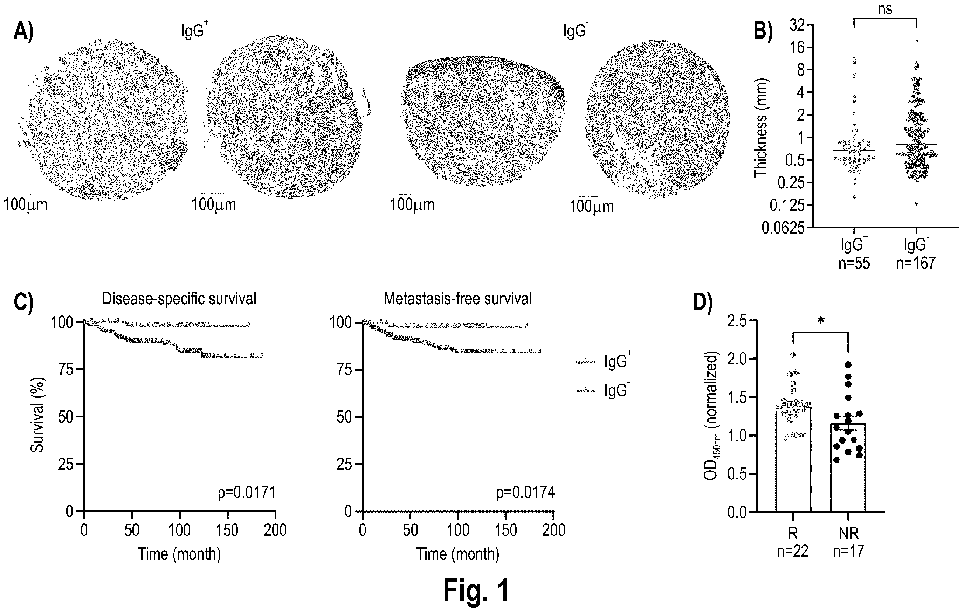

- Fig. 1:: Tumor-infiltrating antibodies in patients with melanoma. (A-C) Melanomas from 222 stage I/II patients were divided into IgG-positive and -negative groups. Representative images of IgG infiltration in both groups (A). Breslow thickness (B) and various survival metrics (C) in both groups. (D) Sera from stage IV melanoma patients responding (R) or not responding (NR) to immune checkpoint inhibitor therapy were tested for TRP2 binding by ELISA.

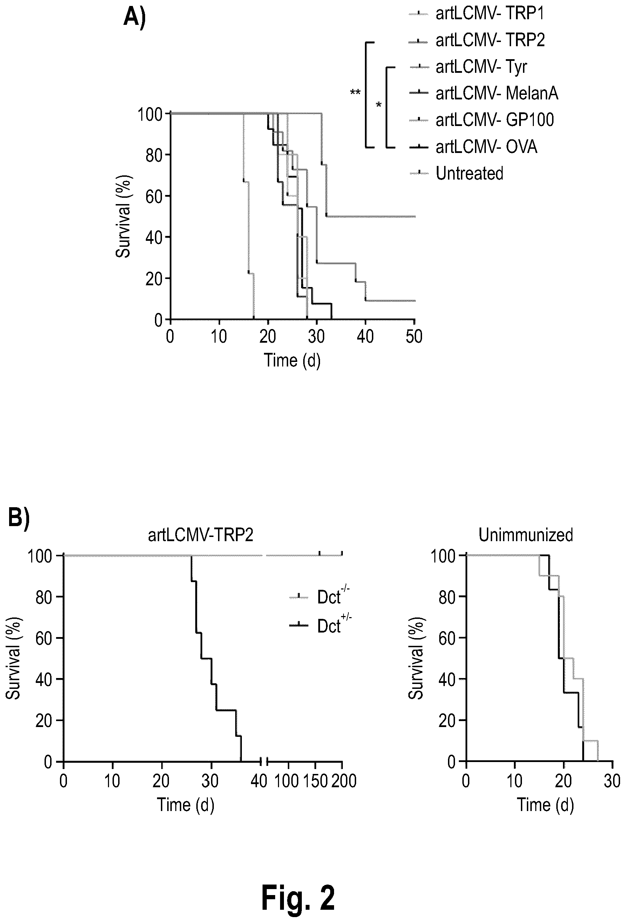

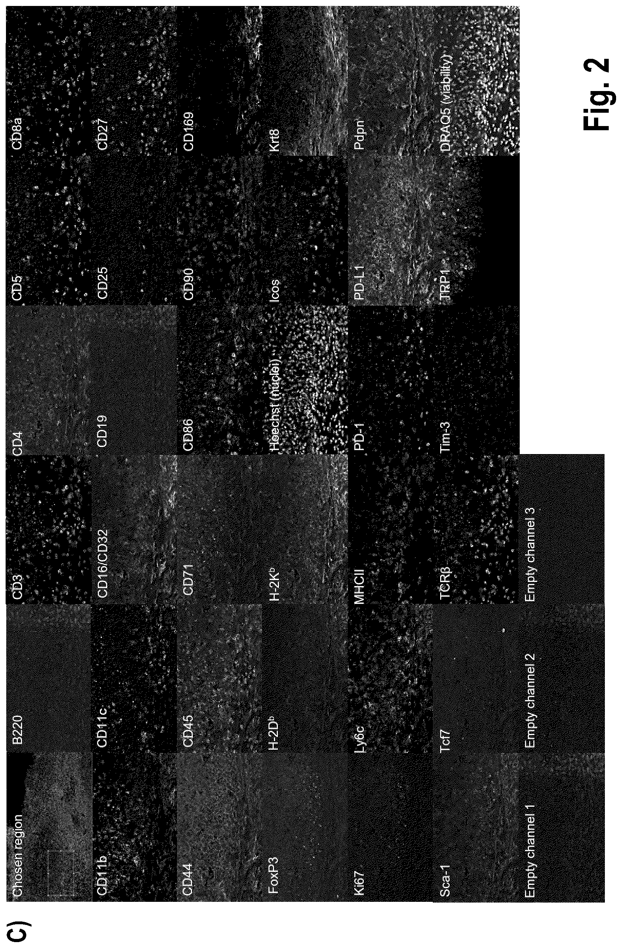

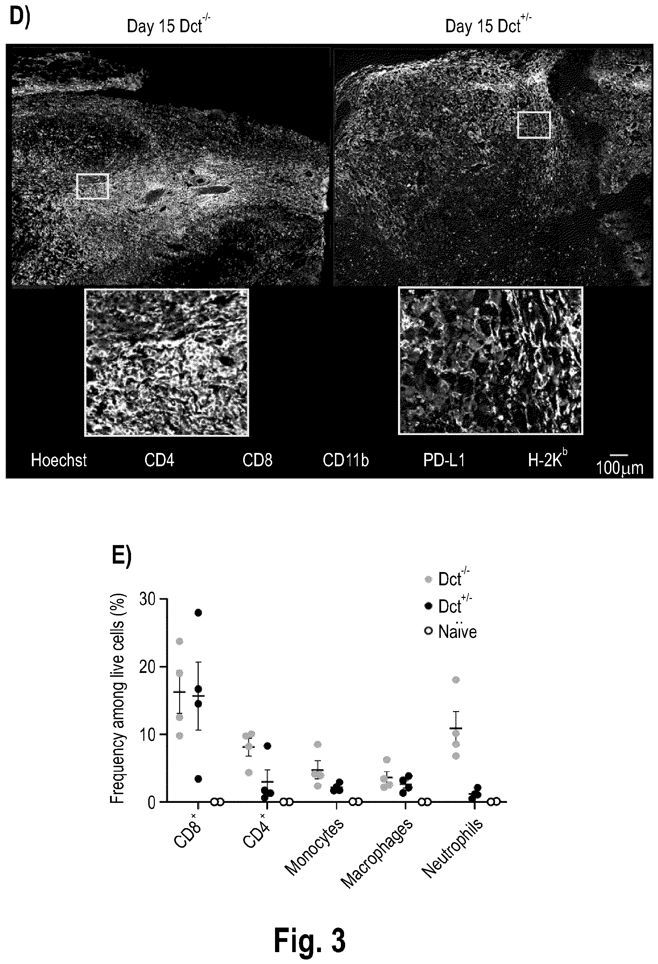

- Fig. 2:: (A) Survival of mice immunized intratumorally with artLCMV vectors expressing different MDAs. (B) Survival of tumor-bearing *Dct*^{-/-} and *Dct*^{+/*-*} mice immunized or not immunized with artLCMV-TRP2. (C) Single-marker images of PhenoCycler staining (see Fig. 3D). MDA - melanocyte differentiation antigen.

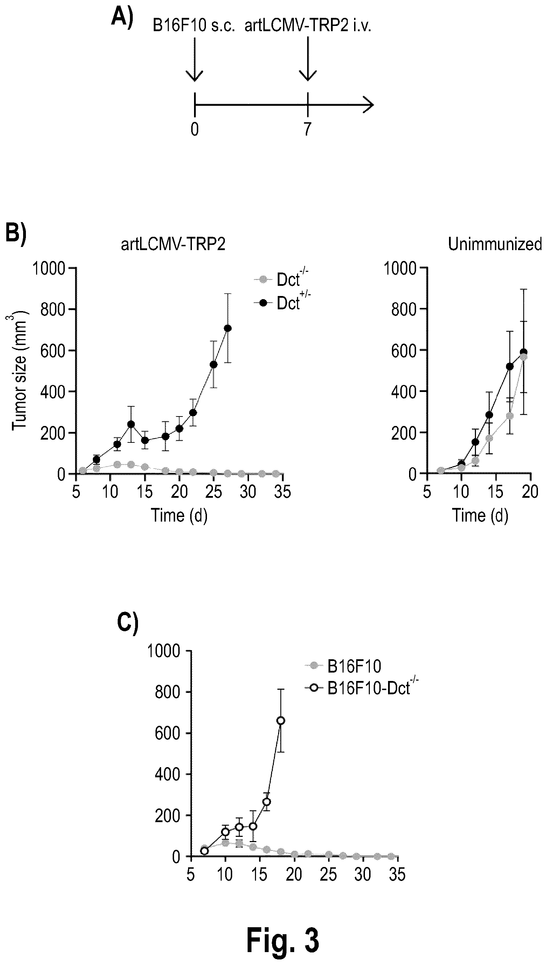

- Fig. 3:: *Dct*^{*-*/*-*} mice TRP2-dependently reject B16F10 melanoma. (A) Experimental setup of tumor growth experiments. Mice were subcutaneously inoculated with B16F10 melanoma cells and intravenously immunized with artLCMV-TRP2 at day 7 of tumor growth. (B) Tumor growth in immunized and unimmunized *Dct*^{-/-} and *Dct*^{+/-} mice. (C) Tumor growth in immunized *Dct*^{-/-} mice inoculated with either wild-type or TRP2-deficient B16F10 cells. (D, E) Tumors from naive and immunized mice were harvested at d15 and stained using PhenoCycler. Representative images of select markers in tumors of immunized *Dct*^{-/-} and *Dct*^{+/-} mice (D) and frequency of various immune cell populations in tumors of naive and immunized mice (E).

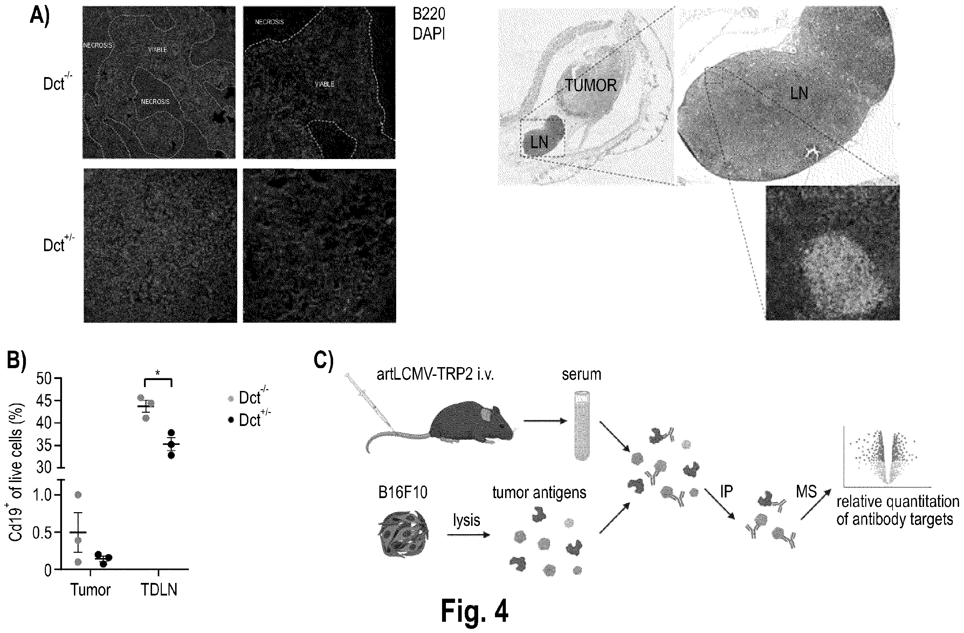

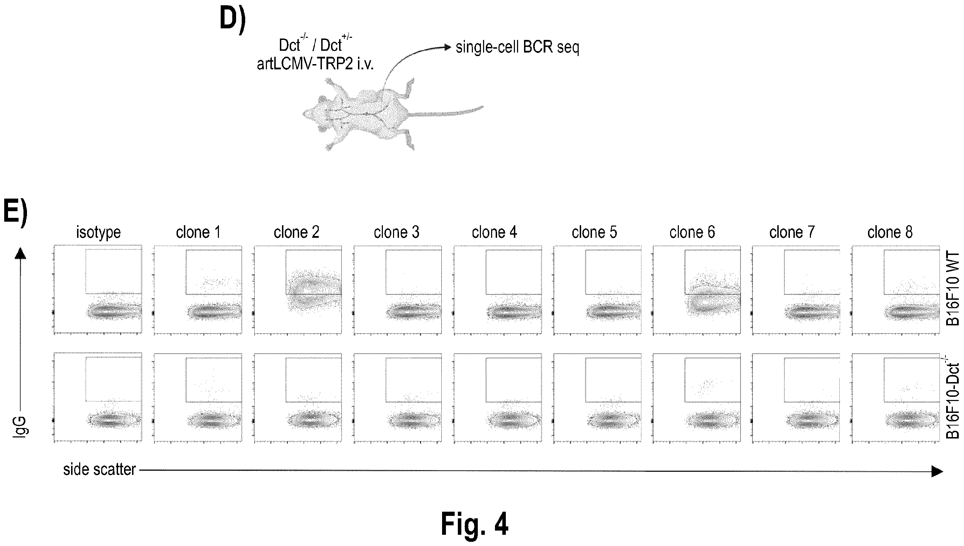

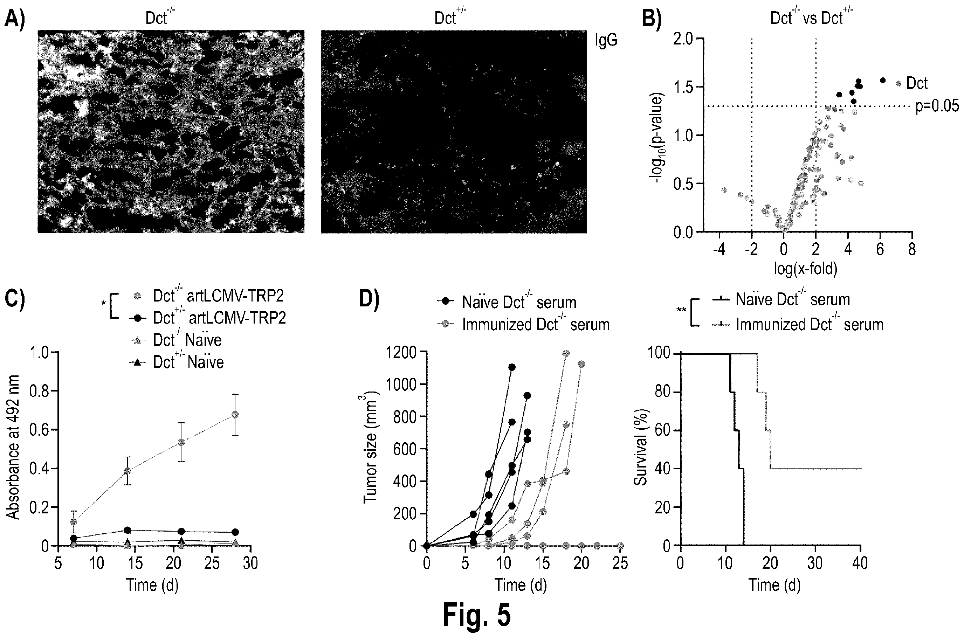

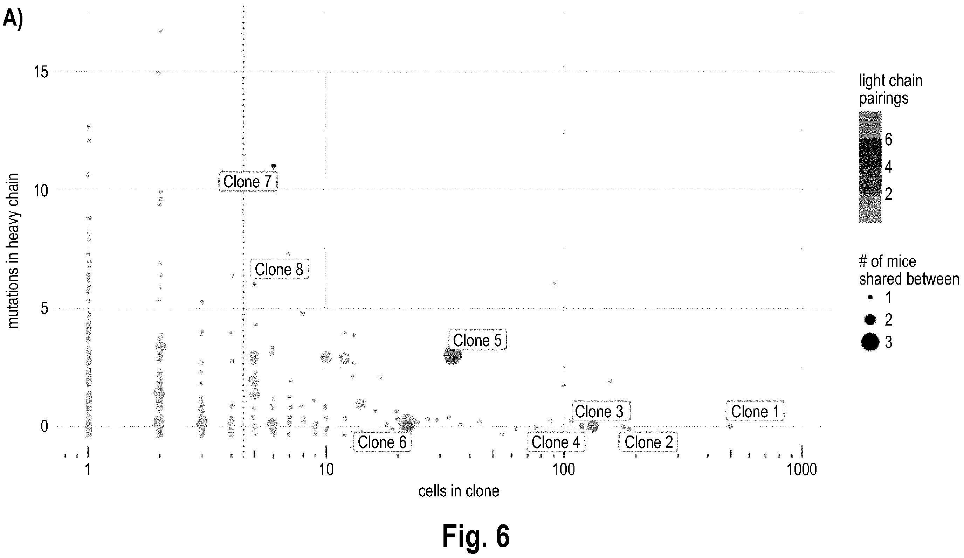

- Fig. 4:: *Dct*^{-/-} and *Dct*^{+/-} mice were inoculated with B16F10 cells, vaccinated with artLCMV-TRP2 at d7 and tumors were collected at d15. Representative images (A) and frequency (B) of B cells in tumor and TDLN. Survival of mice immunized intratumorally with artLCMV vectors expressing different MDAs. (C) Scheme of the IP-MS experiment (see Fig. 5B). *Dct*^{-/-} and *Dct*^{+/-} mice were immunized with artLCMV-TRP2 and sera were collected after 21, 28 and 35 days. Mass spectrometry was performed with serum pulldowns of B16F10 lysates. Created with BioRender.com. (D) Scheme of the single-cell BCR sequencing experiment (see Fig. 6A). Potentially TRP2-binding antibody clones were identified via single-cell BCR sequencing of B cells from tumor-draining lymph nodes of immunized *Dct*^{-/-} and *Dct*^{+/-} mice. Created with BioRender.com. (E) Binding of the antibody clones to wild-type and Dct-deficient B16F10 melanoma cells. BCR - B-cell receptor, IP - immunoprecipitation, MDA - melanocyte differentiation antigen, MS - mass spectrometry, TDLN - tumor-draining lymph node, WT - wild-type.
- Fig. 5:: Immunized *Dct*^{-/-} mice produce protective TRP2-specific antibodies. (A) *Dct*^{*-*/-} and *Dct*^{+/-} mice were inoculated with B16F10 cells, vaccinated with artL-CMV-TRP2 at d7 and tumors were collected at d15. Representative images of IgG antibodies in tumors. (B) *Dct*^{-/-} and *Dct*^{+/-} mice were vaccinated with artLCMV-TRP2 and sera were collected after 21-35 days. Mass spectrometry was performed with serum pulldowns of B16F10 lysate. Volcano plot of enriched targets of *Dct*^{-/-} relative to *Dct*^{+/-} sera. (C) Serum from naive and immunized mice was collected at the indicated timepoints. Binding of IgG in serum (dilution 1:160) to recombinant human TRP2 in ELISA. (D) 300 ul of serum from naïve or immunized *Dct*^{-/-} mice was administered i.p. to WT mice on the day before and on the day of tumor inoculation. Tumor growth in and survival of passively immunized mice.

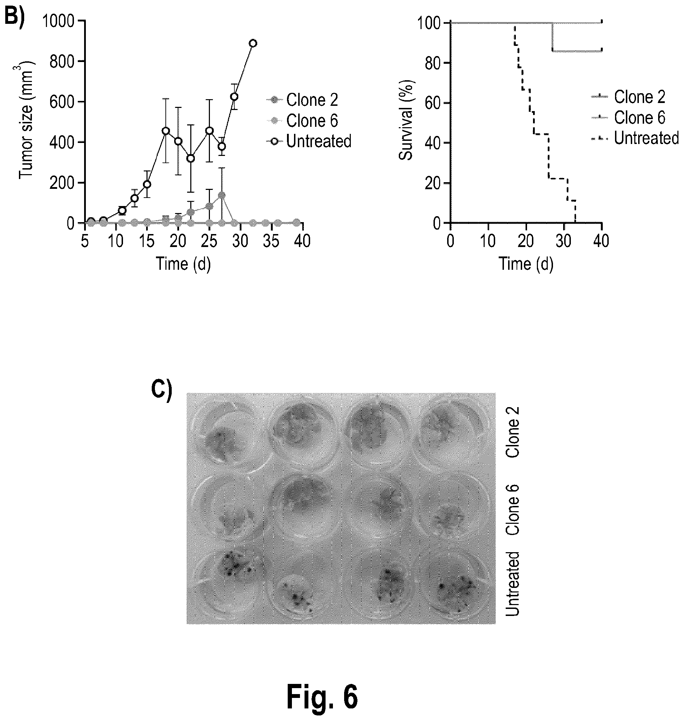

- Fig. 6:: TRP2-specific antibodies protect WT mice in a tumor challenge. (A) Potentially TRP2-binding antibody clones were identified via single-cell BCR sequencing of B cells from tumor-draining lymph nodes of immunized *Dct*^{-/-}and *Dct*^{+/-} mice. Each clone is defined by the heavy chain sequence. Figure depicts the properties based on which the clones were selected: abundance, mutation rate, number of light chain partners and presence in multiple *Dct*^{-/-} mice. (B, C) WT mice were passively immunized with anti-TRP2 antibodies before subcutaneous (B) or intravenous (C) tumor cell inoculation. Growth of subcutaneous tumors and survival of mice (B) and representative images of lung tumor nodules after 14 days (C).

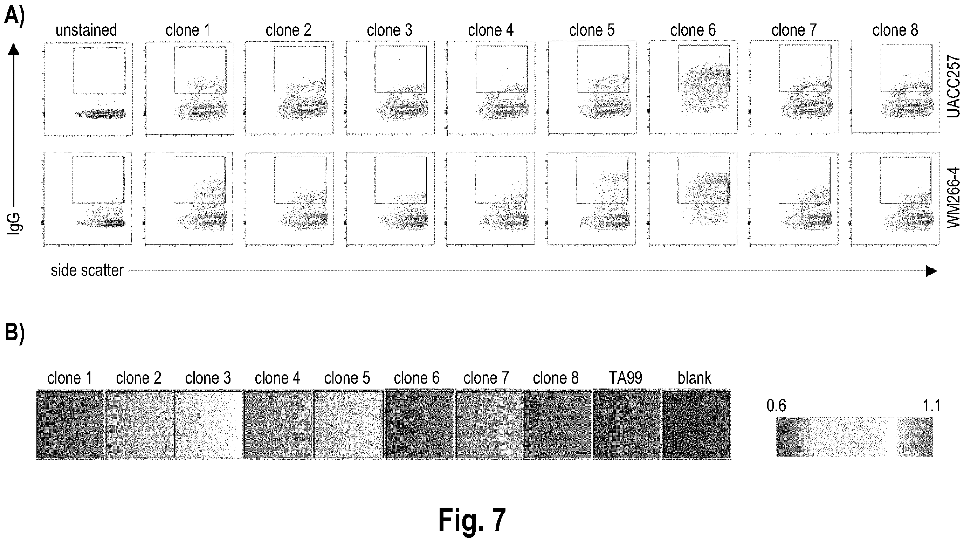

- Fig. 7:: (A) Binding of the antibody clones to TRP2-expressing human melanoma cell lines. (B) Binding of the antibody clones to recombinant human TRP2 in ELISA. TA99 - commercially available anti-TRP1 antibody.

### EXAMPLES

### 1. Introduction

Some melanoma patients go into spontaneous remission, even in advanced metastatic disease. This can be triggered by spontaneous immune responses against melanocyte differentiation antigens (MDAs), which are expressed in melanoma cells and their precursors, normal melanocytes. As such, MDAs are potential targets for melanoma therapies. In this study, the inventors aimed to show a role for antibodies in melanoma and identify MDA-specific antibodies that could be used in adjuvant therapy of melanoma patients. The inventors used mice lacking the MDA tyrosinase-related protein 2 [TRP2; also dopachrome tautomerase (Dct)] to identify TRP2-specific antibody clones, then studied their antitumor efficacy and safety in wild-type (WT) mice.

### 2. Intratumoral antibodies in patients with melanoma

To study the importance of antibodies in melanoma, the inventors first aimed to find antibodies in the tumor. Biopsies of treatment-naive primary melanomas from a cohort of stage I and II patients were analyzed for IgG presence and divided into IgG⁺ and IgG⁻ groups (representative images in Fig. 1A). The groups did not show a significant difference in tumor thickness (Fig. 1B). However, patients with IgG⁺ tumors demonstrated significantly longer disease-specific and metastasis-free survival (Fig. 1C). These results show that the presence of antibodies in the tumor contributes to survival in human melanoma.

In this invention, the inventors are focusing on TRP2 as the prototypic murine MDA: when groups of WT mice were immunized intratumorally with artLCMV vectors encoding five different MDAs, those receiving artLCMV-TRP2 had the greatest survival benefit (Fig. 2A). The inventors have previously shown that antibodies against MDAs, including TRP2, are associated with response to immune checkpoint inhibitors (ICI). The inventors confirmed this in a cohort of 39 stage IV melanoma patients undergoing ICI treatment using sera collected before the start of treatment. Responders to ICI treatment showed significantly higher TRP2 binding than non-responders (Fig. 1D). This suggests that TRP2-specific antibodies may have a protective role in melanoma patients.

### 3. TRP2-dependent tumor rejection in TRP2-deficient mice

To investigate the antitumor effects of therapeutic immunization against TRP2, *Dct*-/- (TRP2 knockout) and *Dct*+/*-* (littermate control) mice were immunized with artLCMV-TRP2 seven days after subcutaneous inoculation of B16F10 melanoma cells (Fig. 3A). *Dct*^{+/-} mice showed a delay in tumor growth, whereas *Dct*^{+/-} mice completely rejected the tumors and survived long-term (Fig. 3B, Fig. 2B). In unimmunized mice, tumor growth and survival times were comparable in both genotypes (Fig. 3B, Fig. 2B). When *Dct*^{+/-} mice were inoculated with TRP2-deficient B16F10-*Dct*^{+/-} tumor cells, they were unable to reject the tumors after artLCMV-TRP2 immunization (Fig. 3C). This indicated that the tumor rejection occurring in immunized *Dct*^{+/-} mice was TRP2-dependent.

To study the differences in immune infiltrate of tumors that may contribute to the opposite outcomes in *Dct*+/- and *Dct*^{+/-} mice, tumors from immunized and naive mice were harvested at day 15 and visualized using PhenoCycler (CODEX), a multiplex immuno¬fluorescence technique. Briefly, tumors were fresh-frozen and cryo¬sectioned, then stained with oligonucleotide-conjugated antibodies against various markers and visualized with complementary, fluorochrome-conjugated oligonucleotides (single-marker stains in Fig. 2C). Tumor infiltration of various immune cells was seen in immunized but not naïve mice (representative images in Fig. 3D).

Quantification of the infiltrating populations revealed some differences between the two groups of immunized mice, most notably the higher frequency of neutrophils in the tumors of *Dct*^{+/-} mice (Fig. 3E). In contrast, the frequency of tumor-infiltrating T cells (both CD8⁺ and CD4⁺) was similar in both genotypes (Fig. 3E), implying that T cells may not be the decisive factor for tumor rejection in this model. However, since tumor rejection in the knockout mice was antigen-dependent, the inventors next investigated the other arm of the adaptive immune system: B cells and antibodies.

### 4. TRP2-specific antibodies in immunized Dct^{-/-} mice

There were few to no tumor-infiltrating B cells in either genotype; however, B cells were found in the tumor-draining (inguinal) lymph nodes (Fig. 4A, B). Moreover, abundant presence of IgG antibodies was observed in tumors of immunized *Dct*^{-/-}mice, whereas very few were seen in *Dct*^{+/-} mice (Fig. 5A). To identify target antigens of the intratumoral antibodies, sera from immunized *Dct*^{-/-} and *Dct*^{+/-} mice were harvested after 21-35 days and incubated with B16F10 cell lysate. Antibodies were precipitated and bound antigens were identified by mass spectrometry (Fig. 4C). TRP2 (Dct) was the most highly enriched target of *Dct*^{-/-} relative to *Dct*^{+/-} serum antibodies (Fig. 5B). It was confirmed by ELISA that *Dct*^{-/-} mice produce significantly more anti-TRP2 IgG antibodies after immunization than *Dct*^{+/-} mice (Fig. 5C).

To see whether serum containing anti-TRP2 antibodies could have a protective antitumor effect in an adjuvant setting, the inventors employed a preventive setting that is similar in its low tumor burden. On the day before and the day of tumor cell inoculation, WT mice were passively immunized with 300 µl of whole serum harvested from naive or immunized *Dct*^{-/-} mice, administered intraperitoneally. Preventive administration of serum from immunized *Dct*^{*-*/*-*} mice delayed tumor growth and improved survival compared with naïve serum, even completely preventing tumor growth in several mice (Fig. 5D). These data demonstrate that immunized *Dct*^{-/-} mice produce TRP2-specific antibodies that recognize tumors and contribute to their rejection in a preventive setting.

### 5. Tumor protective effect of TRP2-specific monoclonal antibodies

To identify TRP2-specific, tumor-reactive antibody clones, single-cell B-cell receptor sequencing was performed on B cells from the tumor-draining lymph node of immunized tumor-bearing *Dct*^{-/-} and *Dct*^{+/-} mice (Fig. 4D). To identify the clones that had the highest likelihood of being TRP2-specific (Fig. 6A), various parameters were taken into account: 1) the presence of the clone only in *Dct*^{*-*/-} and not *Dct*^{+/-} mice; 2) clonal abundance, suggesting clonal expansion after antigen encounter; 3) mutation rate relative to the germline sequence, suggestive of somatic hypermutation; 4) number of different light chain sequences, suggesting high specificity of the heavy chain. Surprisingly, the inventors found B-cell clones that were present in two (clones 3 and 6) or even all three *Dct*^{-/-}mice in the experiment (clone 5). The selected eight clones (Fig. 6A, Table 1, Sequences below) were recombinantly produced as monoclonal antibodies.

**Table 1: Amino acid sequences of the CDRs (complementarity determining regions) of the antibody clones chosen for further testing due to their high likelihood of binding TRP2.**

| **Clone** | **CDR3 heavy chain** | **CDR3 light chain** | **CDR2 heavy chain** | **CDR2 light chain** | **CDR1 heavy chain** | **CDR1 light chain** | **Reason for selection** |
|---|---|---|---|---|---|---|---|
| Clone 1 | ARYYDGYYPLAY (SEQ ID NO: 5) | LQGTHQPYT (SEQ ID NO: 6) | ILPGSGST (SEQ ID NO: 21) | GIS (SEQ ID NO: 22) | GYTFTGYW (SEQ ID NO: 37) | QSLANSYGNTY (SEQ ID NO: 38) | Most abundant clone |
| Clone 2 | ARLGVTTWHGAMDY (SEQ ID NO: 1) | QHHYGTPWT (SEQ ID NO: 2) | ISSGGSYT (SEQ ID NO: 17) | NAK (SEQ ID NO: 18) | GFTFSSYG (SEQ ID NO: 33) | ENIYSY (SEQ ID NO: 34) | Most abundant clone in a different Dct-/- mouse |
| Clone 3 | ARSGGLRWYFDV (SEQ ID NO: 7) | QHSWEIPFT (SEQ ID NO: 8) | IYPGNGDT (SEQ ID NO: 23) | YTS (SEQ ID NO: 24) | GYTFTSYN (SEQ ID NO: 39) | QSVSTSSYSY (SEQ ID NO: 40) | Shared between two *Dct*^{-/-} mice |
| Clone 4 | ARHAKEVYYSNYDYYAMDY (SEQ ID NO: 9) | LQYDNLYT (SEQ ID NO: 10) | FYPGSGSI (SEQ ID NO: 25) | YST (SEQ ID NO: 26) | GYTFTEYT (SEQ ID NO: 41) | ADINKY (SEQ ID NO: 42) | Defined only by the heavy chain |
| Clone 5 | AREGYYGNEGY (SEQ ID NO: 11) | QQGNTLPYT (SEQ ID NO: 12) | ISYDGSN (SEQ ID NO: 27) | YTS (SEQ ID NO: 28) | GYSITSGYY (SEQ ID NO: 43) | QDISNY (SEQ ID NO: 44) | Shared between all three *Dct*^{-/-} mice |
| Clone 6 | ASGAY (SEQ ID NO: 3) | QHFWGTPYT (SEQ ID NO: 4) | ILPGSGST (SEQ ID NO: 19) | AAT (SEQ ID NO: 20) | GYTFTGYW (SEQ ID NO: 35) | ENIYSN (SEQ ID NO: 36) | Shared between two *Dct*^{*-* /-} mice |
| Clone 7 | TRDYGYDDFGV (SEQ ID NO: 13) | QQHYSTPYT (SEQ ID NO: 14) | IDPETGGT (SEQ ID NO: 29) | SAS (SEQ ID NO: 30) | GYIFIDYE (SEQ ID NO: 45) | QDVNTA (SEQ ID NO: 46) | Highest mutation rate of abundant clones |
| Clone 8 | ARAYYDYAYYFDY (SEQ ID NO: 15) | LQSDNMPLT (SEQ ID NO: 16) | IYPGDGDT (SEQ ID NO: 31) | EGN (SEQ ID NO: 32) | GYTFSNYW (SEQ ID NO: 47) | TDIDDD (SEQ ID NO: 46) | Second highest mutation rate of abundant clones |

The eight antibodies were tested for binding to melanoma cells. The TRP2 specificity of the binding to tumor cells was tested by using WT and TRP2-deficient B16F10-*Dct*^{-/-} cells (Fig. 4E). Clones 2 and 6 were chosen for further experiments based on their strong and TRP2-specific binding to melanoma cells. Analogously to serum, 120 µg of the antibodies was administered to WT mice before tumor cell inoculation. Preventive treatment with monoclonal anti-TRP2 antibodies delayed or abolished subcutaneous tumor outgrowth and significantly extended survival (Fig. 6B). In a lung metastasis model, where tumor cells were administered intravenously, preventive antibody treatment significantly reduced the number of tumor nodules in the lungs (representative images in Fig. 6C). Antibody treatment was well tolerated: mice showed no signs of impaired general condition, vitiligo was rare and limited to the tumor area.

Human and murine TRP2 are rather similar, showing an 83.2% amino acid sequence identity, and many commercially available antibodies can detect both species. Thus, the antibodies identified in *Dct*^{-/-} mice were also tested for binding to TRP2-expressing human melanoma cell lines (Fig. 7A). Again, clones 2 and 6 showed strong binding to tumor cells. Binding of the murine antibodies to TRP2 was confirmed by ELISA using recombinant human TRP2 (Fig. 7B). This suggests that these antibodies can also bind human tumor cells and have potential as an adjuvant treatment option in patients with melanoma.

### 6. Conclusion

Tumor-infiltrating B cells and tumor-specific antibodies have been associated with improved prognosis in many tumor types, including melanoma. The results of the inventors also show that intratumoral IgG antibodies are associated with longer disease-specific survival in melanoma patients. This suggests a protective role for antibodies in cancer. Moreover, in patients with HER2-positive breast cancer, the HER2-specific antibody trastuzumab significantly improves survival in the adjuvant setting.

Here the inventors identified potent tumor antigen-specific antibodies with adjuvant application potential using a knock-out mouse. The inventors showed that unlike *Dct*^{+/-} littermate controls, TRP2-deficient *Dct*^{-/-} mice completely rejected B16F10 tumors after therapeutic immunization against TRP2 and their shrinking tumors showed abundant IgG antibodies. The inventors identified TRP2-specific antibody sequences by sequencing B cells from *Dct*^{-/-} mice and showed that those antibodies protected WT mice from a tumor challenge in a preventive, adjuvant-like setting.

Currently, one of the main arguments against adjuvant therapy for all patients with resectable melanoma is the suboptimal cost-benefit balance: many patients are at risk of adverse events without gaining clinical benefit. Monoclonal antibodies targeting a tumor antigen, as opposed to an immune checkpoint, are less toxic while maintaining efficacy. In sum, the novel TRP2-specific antibodies presented here show potential as an alternative, new option for the adjuvant treatment of melanoma.

### Sequences

Part of the application and disclosure is a sequence listing in WIPO St.26-Format. in the following, all sequences are again listed which are subject invention. In the event of discrepancies between the sequences in the attached sequence listing and the sequences listed in the following, the sequences listed in the following shall take precedence.

### AMINO ACIDS

### CDR3

Clone 2 heavy chain CDR3 amino acid
   ARLGVTTWHGAMDY (SEQ ID NO: 1)
Clone 2 light chain CDR3 amino acid
   QHHYGTPWT (SEQ ID NO: 2)
Clone 6 heavy chain CDR3 amino acid
   ASGAY (SEQ ID NO: 3)
Clone 6 light chain CDR3 amino acid
   QHFWGTPYT (SEQ ID NO: 4)
Clone 1 heavy chain CDR3 amino acid
   ARYYDGYYPLAY (SEQ ID NO: 5)
Clone 1 light chain CDR3 amino acid
   LQGTHQPYT (SEQ ID NO: 6)
Clone 3 heavy chain CDR3 amino acid
   ARSGGLRWYFDV (SEQ ID NO: 7)
Clone 3 light chain CDR3 amino acid
   QHSWEIPFT (SEQ ID NO: 8)
Clone 4 heavy chain CDR3 amino acid
   ARHAKEVYYSNYDYYAMDY (SEQ ID NO: 9)
Clone 4 light chain CDR3 amino acid
   LQYDNLYT (SEQ ID NO: 10)
Clone 5 heavy chain CDR3 amino acid
   AREGYYGNEGY (SEQ ID NO: 11)
Clone 5 light chain CDR3 amino acid
   QQGNTLPYT (SEQ ID NO: 12)
Clone 7 heavy chain CDR3 amino acid
   TRDYGYDDFGV (SEQ ID NO: 13)
Clone 7 light chain CDR3 amino acid
   QQHYSTPYT (SEQ ID NO: 14)
Clone 8 heavy chain CDR3 amino acid
   ARAYYDYAYYFDY (SEQ ID NO: 15)
Clone 8 light chain CDR3 amino acid
   LQSDNMPLT (SEQ ID NO: 16)

### CDR2

Clone 2 heavy chain CDR2 amino acid
   ISSGGSYT (SEQ ID NO: 17)
Clone 2 light chain CDR2 amino acid
   NAK (SEQ ID NO: 18)
Clone 6 heavy chain CDR2 amino acid
   ILPGSGST (SEQ ID NO: 19)
Clone 6 light chain CDR2 amino acid
   AAT (SEQ ID NO: 20)
Clone 1 heavy chain CDR2 amino acid
   ILPGSGST (SEQ ID NO: 21)
Clone 1 light chain CDR2 amino acid
   GIS (SEQ ID NO: 22)
Clone 3 heavy chain CDR2 amino acid
   IYPGNGDT (SEQ ID NO: 23)
Clone 3 light chain CDR2 amino acid
   YAS (SEQ ID NO: 24)
Clone 4 heavy chain CDR2 amino acid
   FYPGSGSI (SEQ ID NO: 25)
Clone 4 light chain CDR2 amino acid
   YTS (SEQ ID NO: 26)
Clone 5 heavy chain CDR2 amino acid
   ISYDGSN (SEQ ID NO: 27)
Clone 5 light chain CDR2 amino acid
   YTS (SEQ ID NO: 28)
Clone 7 heavy chain CDR2 amino acid
   IDPETGGT (SEQ ID NO: 29)
Clone 7 light chain CDR2 amino acid
   SAS (SEQ ID NO: 30)
Clone 8 heavy chain CDR2 amino acid
   IYPGDGDT (SEQ ID NO: 31)
Clone 8 light chain CDR2 amino acid
   EGN (SEQ ID NO: 32)

### CDR1

Clone 2 heavy chain CDR1 amino acid
   GFTFSSYG (SEQ ID NO: 33)
Clone 2 light chain CDR1 amino acid
   ENIYSY (SEQ ID NO: 34)
Clone 6 heavy chain CDR1 amino acid
   GYTFTGYW (SEQ ID NO: 35)
Clone 6 light chain CDR1 amino acid
   ENIYSN (SEQ ID NO: 36)
Clone 1 heavy chain CDR1 amino acid
   GYTFTGYW (SEQ ID NO: 37)
Clone 1 light chain CDR1 amino acid
   QSLANSYGNTY (SEQ ID NO: 38)
Clone 3 heavy chain CDR1 amino acid
   GYTFTSYN (SEQ ID NO: 39)
Clone 3 light chain CDR1 amino acid
   QSVSTSSYSY (SEQ ID NO: 40)
Clone 4 heavy chain CDR1 amino acid
   GYTFTEYT (SEQ ID NO: 41)
Clone 4 light chain CDR1 amino acid
   QDINKY (SEQ ID NO: 42)
Clone 5 heavy chain CDR1 amino acid
   GYSITSGYY (SEQ ID NO:43)
Clone 5 light chain CDR1 amino acid
   QDISNY (SEQ ID NO: 44)
Clone 7 heavy chain CDR1 amino acid
   GYIFIDYE (SEQ ID NO: 45)
Clone 7 light chain CDR1 amino acid
   QDVNTA (SEQ ID NO: 46)
Clone 8 heavy chain CDR1 amino acid
   GYTFSNYW (SEQ ID NO: 47)
Clone 8 light chain CDR1 amino acid
   TDIDDD (SEQ ID NO: 48)

### Full sequences

Clone 2 heavy chain full amino acid 
EVQLVESGGDLVKPGGSLKLSCAASGFTFSSYGMSWVRQTPDKRLEWVATISSGGSYTYYPDSVKGRFTISRDNAKNTLYLQMS
SLKSEDTAMYYCARLGVTTVVHGAMDYWGQGTSVTVSS (SEQ ID NO: 49)
Clone 2 light chain full amino acid 
DIQMTQSPASLSASVGETVTITCRASENIYSYLAWYQQKQGKSPQLLVYNAKTLAEGVPSRFSGSGSGTQFSLKINSLQPEDFGSY
YCQHHYGTPWTFGGGTKLEIK (SEQ ID NO: 50)
Clone 6 heavy chain full amino acid 
QVQLQQSGAELMKPGASVKLSCKATGYTFTGYWIEWVKQRPGHGLEWIGEILPGSGSTNYNEKFKGKATFTADTSSNTAYMQLS
SLTTEDSAIYYCASGAYWGQGTLVTVSA (SEQ ID NO: 51)
Clone 6 light chain full amino acid 
DIQMTQSPASLSVSVGETVTITCRASENIYSNLAWYQQKQGKSPQLLVYAATNLADGVPSRFSGSGSGTQYSLKINSLQSEDFGS
YYCQHFWGTPYTFGGGTKLEIK (SEQ ID NO: 52)
Clone 1 heavy chain full amino acid 
QVQLQQSGAELMKPGASVKLSCKATGYTFTGYWIEWVKQRPGHGLEWIGEILPGSGSTNYNEKFKGKATFTADTSSNTAYMQLS
SLTTEDSAIYYCARYYDGYYPLAYWGQGTLVTVSA (SEQ ID NO: 53)
Clone **1** light chain full amino acid 
DVVVTQTPLSLPVSFGDQVSISCRSSQSLANSYGNTYLSWYLHKPGQSPQLLIYGISNRFSGVPDRFSGSGSGTDFTLKISTIKPED
LGMYYCLQGTHQPYTFGGGTKLEIK (SEQ ID NO: 54)
Clone 3 heavy chain full amino acid 
QAYLQQSGAELVRPGASVKMSCKASGYTFTSYNMHWVKQTPRQGLEWIGAIYPGNGDTSYNQKFKGKATLTVDKSSSTAYMQL
SSLTSEDSAVYFCARSGGLRWYFDVWGTGTTVTVSS (SEQ ID NO: 55)
Clone 3 light chain full amino acid 
DIVLTQSPASLAVSLGQRATISCRASQSVSTSSYSYMHWYQQKPGQPPKLLIKYASNLESGVPARFSGSGSGTDFTLNIHPVEEED
TATYYCQHSWEIPFTFGSGTKLEIK (SEQ ID NO: 56)
Clone 4 heavy chain full amino acid 
QVQLQQSGAELVKPGASVKLSCKASGYTFTEYTIHWVKQRSGQGLEWIGWFYPGSGSIKYNEKFKDKATLTADKSSSTVYMELS
RLTSEDSAVYFCARHAKEVYYSNYDYYAMDYWGQGTSVTVSS (SEQ ID NO: 57)
**Clone 4** light chain full amino acid 
DIQMTQSPSSLSASLGGKVTITCKASQDINKYIAWYQHKPGKGPRLLIHYTSTLQPGIPSRFSGSGSGRDYSFSISNLEPEDIATYYC
LQYDNLYTFGGGTKLEIK (SEQ ID NO: 58)
Clone 5 heavy chain full amino acid 
DVQLQESGPGLVKPSQSLSLTCSVTGYSITSGYYWNWIRQFPGNKLEWMGYISYDGSNNYNPSLKNRISITRDTSKNQFFLKLNS
VTTEDTATYYCAREGYYGNEGYWGQGTTLTVSS (SEQ ID NO: 59)
Clone 5 light chain full amino acid 
DIQMTQTTSSLSASLGDRVTISCRASQDISNYLNWYQQKPDGTVKLLIYYTSRLHSGVPSRFSGSGSGTDYSLTISNLEQEDIATYF
CQQGNTLPYTFGGGTKLEIK (SEQ ID NO: 60)
Clone 7 heavy chain full amino acid 
QVQLQQSGAELVRPGASVTLSCKAPGYIFIDYEIHWVKQTPVHGLEWIGAIDPETGGTAYNQKFKGKARLTADISSSTAYMDLRSL
TSEDSAVYYCTRDYGYDDFGVWGTGTTVTVSS (SEQ ID NO: 61)
**Clone 7 light chain** full **amino** acid 
DIVMTQSHKFMSTSVGDRVNITCKASQDVNTAVAWYQQAPGQSPKLLIYSASYRYTGVPDRFTGSGSGTDFTFTISSVKAEDLAV
YYCQQHYSTPYTFGGGTKLEIK (SEQ ID NO: 62)
Clone 8 heavy chain full amino acid 
QVQLQQSGAELVKPGASVKISCKASGYTFSNYWMNWVKQRPGKGLQWIGQIYPGDGDTNYNGKFEGTATLTADKSSSTAYMQL
SSLTSEDSAVYFCARAYYDYAYYFDYWGQGTTLTVSS (SEQ ID NO: 63)
**Clone 8 light chain** full **amino** acid

ETTVTQSPASLSVATGEKVTIRCIISTDIDDDMNWYQQKPGEPPKLLISEGNTLRPGVPSRFSSSGYGTDFVFTIENTLSEDVADYY
CLQSDNMPLTFGGGTKLEIK (SEQ ID NO: 64)

### FRs

Clone 2 heavy chain FR1 amino acid
   EVQLVESGGDLVKPGGSLKLSCAAS (SEQ ID NO: 65)
Clone 2 heavy chain FR2 amino acid
   MSWVRQTPDKRLEWVAT (SEQ ID NO: 66)
Clone 2 heavy chain FR3 amino acid
   YYPDSVKGRFTISRDNAKNTLYLQMSSLKSEDTAMYYC (SEQ ID NO: 67)
Clone 2 heavy chain FR4 amino acid
   WGQGTSVTVSS (SEQ ID NO: 68)
Clone 2 light chain FR1 amino acid
   DIQMTQSPASLSASVGETVTITCRAS (SEQ ID NO: 69)
Clone 2 light chain FR2 amino acid
   LAWYQQKQGKSPQLLVY (SEQ ID NO: 70)
Clone 2 light chain FR3 amino acid
   TLAEGVPSRFSGSGSGTQFSLKINSLQPEDFGSYYC (SEQ ID NO: 71)
Clone 2 light chain FR4 amino acid
   FGGGTKLElK (SEQ ID NO: 72)
Clone 6 heavy chain FR1 amino acid
   QVQLQQSGAELMKPGASVKLSCKAT (SEQ ID NO: 73)
Clone 6 heavy chain FR2 amino acid
   IEWVKQRPGHGLEWIGE (SEQ ID NO: 74)
Clone 6 heavy chain FR3 amino acid
   NYNEKFKGKATFTADTSSNTAYMQLSSLTTEDSAIYYC (SEQ ID NO: 75)
Clone 6 heavy chain FR4 amino acid
   WGaGTLVTVSA (SEQ ID NO: 76)
Clone 6 light chain FR1 amino acid
   DIQMTQSPASLSVSVGETVTITCRAS (SEQ ID NO: 77)
Clone 6 light chain FR2 amino acid
   LAWYQQKQGKSPQLLVY (SEQ ID NO: 78)
Clone 6 light chain FR3 amino acid
   NLADGVPSRFSGSGSGTQYSLKINSLQSEDFGSYYC (SEQ ID NO: 79)
Clone 6 light chain FR4 amino acid
   FGGGTKLElK (SEQ ID NO: 80)
Clone 1 heavy chain FR1 amino acid
   QVQLQQSGAELMKPGASVKLSCKAT (SEQ ID NO: 81)
Clone 1 heavy chain FR2 amino acid
   IEWVKQRPGHGLEWIGE (SEQ ID NO: 82)
Clone 1 heavy chain FR3 amino acid
   NYNEKFKGKATFTADTSSNTAYMQLSSLTTEDSAIYYC (SEQ ID NO: 83)
Clone 1 heavy chain FR4 amino acid
   WGaGTLVTVSA (SEQ ID NO: 84)
Clone 1 light chain FR1 amino acid
   DWVTQTPLSLPVSFGDQVSiSCRSS (SEQ ID NO: 85)
Clone 1 light chain FR2 amino acid
   LSWYLHKPGQSPQLLIY (SEQ ID NO: 88)
Clone 1 light chain FR3 amino acid
   NRFSGVPDRFSGSGSGTDFTLKISTIKPEDLGMYYC (SEQ ID NO: 87)
Clone 1 light chain FR4 amino acid
   FGGGTKLElK (SEQ ID NO: 88)
Clone 3 heavy chain FR1 amino acid
   QAYLQQSGAELVRPGASVKMSCKAS (SEQ ID NO: 89)
Clone 3 heavy chain FR2 amino acid
   MHWVKQTPRQGLEWIGA (SEQ ID NO: 90)
Clone 3 heavy chain FR3 amino acid
   SYNQKFKGKATLTVDKSSSTAYMQLSSLTSEDSAVYFC (SEQ ID NO: 91)
Clone 3 heavy chain FR4 amino acid
   WGTGTTVTVSS (SEQ ID NO: 92)
Clone 3 light chain FR1 amino acid
   DIVLTQSPASLAVSLGQRATISCRAS (SEQ ID NO: 93)
Clone 3 light chain FR2 amino acid
   MHWYQQKPGQPPKLLIK (SEQ ID NO: 94)
Clone 3 light chain FR3 amino acid
   NLESGVPARFSGSGSGTDFTLNIHPVEEEDTATYYC (SEQ ID NO: 95)
Clone 3 light chain FR4 amino acid
   FGSGTKLEIK (SEQ ID NO: 96)
Clone 4 heavy chain FR1 amino acid
   QVQLQQSGAELVKPGASVKLSCKAS (SEQ ID NO: 97)
Clone 4 heavy chain FR2 amino acid
   IHWVKQRSGQGLEWIGW (SEQ ID NO: 98)
Clone 4 heavy chain FR3 amino acid
   KYNEKFKDKATLTADKSSSTVYMELSRLTSEDSAVYFC (SEQ ID NO: 99)
Clone 4 heavy chain FR4 amino acid
   WGQGTSVTVSS (SEQ ID NO: 100)
Clone 4 light chain FR1 amino acid
   DIQMTQSPSSLSASLGGKVTITCKAS (SEQ ID NO: 101)
Clone 4 light chain FR2 amino acid
   IAWYQHKPGKGPRLLIH (SEQ ID NO: 102)
Clone 4 light chain FR3 amino acid
   TLQPGIPSRFSGSGSGRDYSFSISNLEPEDIATYYC (SEQ ID NO: 103)
Clone 4 light chain FR4 amino acid
   FGGGTKLElK (SEQ ID NO: 104)
Clone 5 heavy chain FR1 amino acid
   DVQLQESGPGLVKPSQSLSLTCSVT (SEQ ID NO: 105)
Clone 5 heavy chain FR2 amino acid
   WNWIRQFPGNKLEWMGY (SEQ ID NO: 108)
Clone 5 heavy chain FR3 amino acid
   NYNPSLKNRISITRDTSKNQFFLKLNSVTTEDTATYYC (SEQ ID NO: 107)
Clone 5 heavy chain FR4 amino acid
   WGQGTTLTVSS (SEQ ID NO: 108)
Clone 5 light chain FR1 amino acid
   DIQMTQTTSSLSASLGDRVTISCRAS (SEQ ID NO: 109)
Clone 5 light chain FR2 amino acid
   LNWYQQKPDGTVKLLIY (SEQ ID NO: 110)
Clone 5 light chain FR3 amino acid
   RLHSGVPSRFSGSGSGTDYSLTISNLEQEDIATYFC (SEQ ID NO: 111)
Clone 5 light chain FR4 amino acid
   FGGGTKLElK (SEQ ID NO: 112)
Clone 7 heavy chain FR1 amino acid
   QVQLQQSGAELVRPGASVTLSCKAP (SEQ ID NO: 113)
Clone 7 heavy chain FR2 amino acid
   IHWVKQTPVHGLEWIGA (SEQ ID NO: 114)
Clone 7 heavy chain FR3 amino acid
   AYNQKFKGKARLTADISSSTAYMDLRSLTSEDSAVYYC (SEQ ID NO: 115)
Clone 7 heavy chain FR4 amino acid
   WGTGTTVTVSS (SEQ ID NO: 116)
Clone 7 light chain FR1 amino acid
   DIVMTQSHKFMSTSVGDRVNITCKAS (SEQ ID NO: 117)
Clone 7 light chain FR2 amino acid
   VAWYQQAPGQSPKLLIY (SEQ ID NO: 118)
Clone 7 light chain FR3 amino acid
   YRYTGVPDRFTGSGSGTDFTFTISSVKAEDLAVYYC (SEQ ID NO: 119)
Clone 7 light chain FR4 amino acid
   FGGGTKLElK (SEQ ID NO: 120)
Clone 8 heavy chain FR1 amino acid
   QVQLQQSGAELVKPGASVKISCKAS (SEQ ID NO: 121)
Clone 8 heavy chain FR2 amino acid
   MNWVKQRPGKGLQWIGQ (SEQ ID NO: 122)
Clone 8 heavy chain FR3 amino acid
   NYNGKFEGTATLTADKSSSTAYMQLSSLTSEDSAVYFC (SEQ ID NO: 123)
Clone 8 heavy chain FR4 amino acid
   WGQGTTLTVSS (SEQ ID NO: 124)
Clone 8 light chain FR1 amino acid
   ETTVTQSPASLSVATGEKVTIRCIIS (SEQ ID NO: 125)
Clone 8 light chain FR2 amino acid
   MNWYQQKPGEPPKLLIS (SEQ ID NO: 126)
Clone 8 light chain FR3 amino acid
   TLRPGVPSRFSSSGYGTDFVFTIENTLSEDVADYYC (SEQ ID NO: 127)
Clone 8 light chain FR4 amino acid
   FGGGTKLElK (SEQ ID NO: 128)

### NUCLEIC ACIDS

Clone 2 heavy chain full length nucleic acid, incl. FR1-CDR1-FR2-CDR2-FR3-CDR3-FR4 
GAGGTGCAGCTGGTGGAGTCTGGGGGAGACTTAGTGAAGCCTGGAGGGTCCCTGAAACTCTCCTGTGCAGCCTCTGGATT
CACTTTCAGTAGCTATGGCATGTCTTGGGTTCGCCAGACTCCAGACAAGAGGCTGGAGTGGGTCGCAACCATTAGTAGTGG
TGGTAGTTACACCTACTATCCAGACAGTGTGAAGGGGCGATTCACCATCTCCAGAGACAATGCCAAGAACACCCTGTACCTG
CAAATGAGCAGTCTGAAGTCTGAGGACACAGCCATGTATTACTGTGCAAGACTGGGGGTTACTACGGTAGTACACGGTGCTA
TGGACTACTGGGGTCAAGGAACCTCAGTCACCGTCTCCTCAG (SEQ ID NO: 129)
Clone 2 light chain full length nucleic acid, incl. FR1-CDR1-FR2-CDR2-FR3-CDR3-FR4 
GACATCCAGATGACTCAGTCTCCAGCCTCCCTATCTGCATCTGTGGGAGAAACTGTCACCATCACATGTCGAGCAAGTGAGA
ATATTTACAGTTATTTAGCATGGTATCAGCAGAAACAGGGAAAATCTCCTCAGCTCCTGGTCTATAATGCAAAAACCTTAGCA
GAAGGTGTGCCATCAAGGTTCAGTGGCAGTGGATCAGGCACACAGTTTTCTCTGAAGATCAACAGCCTGCAGCCTGAAGATT
TTGGGAGTTATTACTGTCAACATCATTATGGTACTCCGTGGACGTTCGGTGGAGGCACCAAGCTGGAAATCAAAC
(SEQ ID NO: 130)
Clone 6 heavy chain full length nucleic acid, incl. FR1-CDR1-FR2-CDR2-FR3-CDR3-FR4 
CAGGTTCAGCTGCAGCAGTCTGGAGCTGAGCTGATGAAGCCTGGGGCCTCAGTGAAGCTTTCCTGCAAGGCTACTGGCTAC
ACATTCACTGGCTACTGGATAGAGTGGGTAAAGCAGAGGCCTGGACATGGCCTTGAGTGGATTGGAGAGATTTTACCTGGA
AGTGGTAGTACTAACTACAATGAGAAGTTCAAGGGCAAGGCCACATTCACTGCAGATACATCCTCCAACACAGCCTACATGC
AACTCAGCAGCCTGACAACTGAGGACTCTGCCATCTATTACTGTGCAAGTGGTGCTTACTGGGGCCAAGGGACTCTGGTCA
CTGTCTCTGCAG (SEQ ID NO: 131)
Clone 6 light chain full length nucleic acid, incl. FR1-CDR1-FR2-CDR2-FR3-CDR3-FR4 
GACATCCAGATGACTCAGTCTCCAGCCTCCCTATCTGTATCTGTGGGAGAAACTGTCACCATCACATGTCGAGCAAGTGAGA
ATATTTACAGTAATTTAGCATGGTATCAGCAGAAACAGGGAAAATCTCCTCAGCTCCTGGTCTATGCTGCAACAAACTTAGCA
GATGGTGTGCCATCAAGGTTCAGTGGCAGTGGATCAGGCACACAGTATTCCCTCAAGATCAACAGCCTGCAGTCTGAAGATT
TTGGGAGTTATTACTGTCAACATTTTTGGGGTACTCCGTACACGTTCGGAGGGGGGACCAAGCTGGAAATAAAC (SEQ ID
NO: 132)
Clone 1 heavy chain full length nucleic acid, incl. FR1-CDR1-FR2-CDR2-FR3-CDR3-FR4 
CAGGTTCAGCTGCAGCAGTCTGGAGCTGAGCTGATGAAGCCTGGGGCCTCAGTGAAGCTTTCCTGCAAGGCTACTGGCTAC
ACATTCACTGGCTACTGGATAGAGTGGGTAAAGCAGAGGCCTGGACATGGCCTTGAGTGGATTGGAGAGATTTTACCTGGA
AGTGGTAGTACTAACTACAATGAGAAGTTCAAGGGCAAGGCCACATTCACTGCAGATACATCCTCCAACACAGCCTACATGC
AACTCAGCAGCCTGACAACTGAGGACTCTGCCATCTATTACTGTGCAAGATACTATGATGGTTACTACCCTTTGGCTTACTGG
GGCCAAGGGACTCTGGTCACTGTCTCTGCAG (SEQ ID NO: 133)
Clone 1 light chain full length nucleic acid, incl. FR1-CDR1-FR2-CDR2-FR3-CDR3-FR4 
GATGTTGTGGTGACTCAAACTCCACTCTCCCTGCCTGTCAGCTTTGGAGATCAAGTTTCTATCTCTTGCAGGTCTAGTCAGAG
TCTTGCAAACAGTTATGGGAACACCTATTTGTCTTGGTACCTGCACAAGCCTGGCCAGTCTCCACAGCTCCTCATCTATGGG
ATTTCCAACAGATTTTCTGGGGTGCCAGACAGGTTCAGTGGCAGTGGTTCAGGGACAGATTTCACACTCAAGATCAGCACAA
TAAAGCCTGAGGACTTGGGAATGTATTACTGCTTACAAGGTACACATCAGCCGTACACGTTCGGAGGGGGGACCAAGCTGG
AAATAAAAC (SEQ ID NO: 134)
Clone 3 heavy chain full length nucleic acid, incl. FR1-CDR1-FR2-CDR2-FR3-CDR3-FR4 
CAGGCTTATCTACAGCAGTCTGGGGCTGAGCTGGTGAGGCCTGGGGCCTCAGTGAAGATGTCCTGCAAGGCTTCTGGCTAC
ACATTTACCAGTTACAATATGCACTGGGTAAAGCAGACACCTAGACAGGGCCTGGAATGGATTGGAGCTATTTATCCAGGAA
ATGGTGATACTTCCTACAATCAGAAGTTCAAGGGCAAGGCCACACTGACTGTAGACAAATCCTCCAGCACAGCCTACATGCA
GCTCAGCAGCCTGACATCTGAAGACTCTGCGGTCTATTTCTGTGCAAGATCGGGGGGATTACGTTGGTACTTCGATGTCTGG
GGCACAGGGACCACGGTCACCGTCTCCTCAG (SEQ ID NO: 135)
Clone 3 light chain full length nucleic acid, incl. FR1-CDR1-FR2-CDR2-FR3-CDR3-FR4 
GACATTGTGCTGACACAGTCTCCTGCTTCCTTAGCTGTATCTCTGGGGCAGAGGGCCACCATCTCATGCAGGGCCAGCCAA
AGTGTCAGTACATCTAGCTATAGTTATATGCACTGGTACCAACAGAAACCAGGACAGCCACCCAAACTCCTCATCAAGTATGC
ATCCAACCTAGAATCTGGGGTCCCTGCCAGGTTCAGTGGCAGTGGGTCTGGGACAGACTTCACCCTCAACATCCATCCTGT
GGAGGAGGAGGATACTGCAACATATTACTGTCAGCACAGTTGGGAGATTCCATTCACGTTCGGCTCGGGGACAAAGTTGGA
AATAAAAC (SEQ ID NO: 136)
Clone 4 heavy chain full length nucleic acid, incl. FR1-CDR1-FR2-CDR2-FR3-CDR3-FR4 
CAGGTCCAGCTGCAGCAGTCTGGAGCTGAGCTGGTGAAACCCGGGGCATCAGTGAAGCTGTCCTGCAAGGCTTCTGGCTA
CACCTTCACTGAGTATACTATACACTGGGTAAAGCAGAGGTCTGGACAGGGTCTTGAGTGGATTGGGTGGTTTTACCCTGGA
AGTGGTAGTATAAAGTACAATGAGAAATTCAAGGACAAGGCCACATTGACTGCGGACAAATCCTCCAGCACAGTCTATATGG
AGCTTAGTAGATTGACATCTGAAGACTCTGCGGTCTATTTCTGTGCAAGACACGCGAAGGAGGTCTACTATAGTAACTACGAT
TACTATGCTATGGACTACTGGGGTCAAGGAACCTCAGTCACCGTCTCCTCAG (SEQ ID NO: 137)
Clone 4 light chain full length nucleic acid, incl. FR1-CDR1-FR2-CDR2-FR3-CDR3-FR4 
GACATCCAGATGACACAGTCTCCATCCTCACTGTCTGCATCTCTGGGAGGCAAAGTCACCATCACTTGCAAGGCAAGCCAAG
ACATTAACAAGTATATAGCTTGGTACCAACACAAGCCTGGAAAAGGTCCTAGGCTGCTCATACATTACACATCTACATTACAG
CCAGGCATCCCATCAAGGTTCAGTGGAAGTGGGTCTGGGAGAGATTATTCCTTCAGCATCAGCAACCTGGAGCCTGAAGAT
ATTGCAACTTATTATTGTCTACAGTATGATAATCTGTACACGTTCGGAGGGGGGACCAAGCTGGAAATAAAAC (SEQ ID NO:
138)
Clone 5 heavy chain full length nucleic acid, incl. FR1-CDR1-FR2-CDR2-FR3-CDR3-FR4 
GATGTACAGCTTCAGGAGTCAGGACCTGGCCTCGTGAAACCTTCTCAGTCTCTGTCTCTCACCTGCTCTGTCACTGGCTACT
CCATCACCAGTGGTTATTACTGGAACTGGATCCGGCAGTTTCCAGGAAACAAACTGGAATGGATGGGCTACATAAGCTACGA
TGGTAGCAATAACTACAACCCATCTCTCAAAAATCGAATCTCCATCACTCGTGACACATCTAAGAACCAGTTTTTCCTGAAGTT
GAATTCTGTGACTACTGAGGACACAGCCACATATTACTGTGCAAGAGAGGGCTACTATGGTAACGAAGGCTACTGGGGCCA
AGGCACCACTCTCACAGTCTCCTCAG (SEQ ID NO: 139)
Clone 5 **light** chain full length nucleic acid, incl. FR1-CDR1-FR2-CDR2-FR3-CDR3-FR4 
GATATCCAGATGACACAGACTACATCCTCCCTGTCTGCCTCTCTGGGAGACAGAGTCACCATCAGTTGCAGGGCAAGTCAG
GACATTAGCAATTATTTAAACTGGTATCAGCAGAAACCAGATGGAACTGTTAAACTCCTGATCTACTACACATCAAGATTACAC
TCAGGAGTCCCATCAAGGTTCAGTGGCAGTGGGTCTGGAACAGATTATTCTCTCACCATTAGCAACCTGGAGCAAGAAGATA
TTGCCACTTACTTTTGCCAACAGGGTAATACGCTTCCGTACACGTTCGGAGGGGGGACCAAGCTGGAAATAAAAC (SEQ ID
NO: 140)
Clone 7 heavy chain full length nucleic acid, incl. FR1-CDR1-FR2-CDR2-FR3-CDR3-FR4 
CAGGTTCAACTGCAGCAGTCTGGGGCTGAGCTGGTGAGGCCTGGGGCTTCAGTGACGCTGTCCTGCAAGGCTCCGGGCTA
CATATTTATTGACTATGAAATACACTGGGTGAAGCAGACACCTGTGCATGGCCTGGAATGGATTGGAGCTATTGATCCTGAAA
CTGGAGGTACTGCCTACAATCAGAAGTTCAAGGGCAAGGCCAGACTGACTGCAGACATATCCTCCAGTACAGCCTACATGG
ACCTCCGCAGCCTGACATCTGAGGACTCTGCCGTCTATTACTGTACAAGAGACTATGGTTACGACGACTTTGGTGTCTGGGG
CACAGGGACCACGGTCACCGTCTCCTCAG (SEQ ID NO: 141)
Clone 7 **light** chain full length nucleic acid, incl. FR1-CDR1-FR2-CDR2-FR3-CDR3-FR4 
GACATTGTGATGACCCAGTCTCACAAATTCATGTCCACATCAGTAGGAGACAGGGTCAACATCACCTGCAAGGCCAGTCAGG
ATGTGAATACTGCTGTAGCCTGGTATCAACAGGCTCCAGGACAATCTCCTAAACTACTGATTTACTCGGCATCCTACCGGTAC
ACTGGAGTCCCTGATCGCTTCACTGGCAGTGGATCTGGGACGGATTTCACTTTCACCATCAGCAGTGTGAAGGCTGAAGAC
CTGGCAGTTTATTACTGTCAGCAACATTATAGTACTCCGTACACGTTCGGAGGGGGGACCAAGCTGGAAATAAAAC (SEQ ID
NO: 142)
Clone 8 heavy chain full length nucleic acid, incl. FR1-CDR1-FR2-CDR2-FR3-CDR3-FR4 
CAGGTTCAGCTGCAGCAGTCTGGGGCTGAGCTGGTGAAGCCTGGGGCCTCAGTGAAGATTTCCTGCAAAGCTTCTGGCTAC
ACATTCAGTAACTACTGGATGAACTGGGTGAAGCAGAGGCCTGGAAAGGGTCTTCAATGGATTGGACAGATTTATCCTGGAG
ATGGTGATACTAACTACAACGGAAAGTTCGAGGGCACGGCCACACTGACTGCAGACAAATCCTCCAGCACAGCCTACATGC
AGCTCAGCAGCCTGACCTCTGAGGACTCTGCGGTCTATTTCTGTGCAAGAGCCTACTATGATTACGCCTACTACTTTGACTA
CTGGGGCCAAGGCACCACTCTCACAGTCTCCTCAG (SEQ ID NO: 143)
Clone 8 light chain full length nucleic acid, incl. FR1-CDR1-FR2-CDR2-FR3-CDR3-FR4

GAAACAACTGTGACCCAGTCTCCAGCGTCCCTGTCCGTGGCTACAGGAGAAAAAGTCACTATCAGATGCATAATCAGCACTG
ATATTGATGATGATATGAACTGGTACCAGCAGAAGCCAGGGGAACCTCCTAAACTCCTTATTTCAGAAGGCAATACTCTTCGT
CCTGGAGTCCCATCCCGATTCTCCAGCAGTGGCTATGGCACAGATTTTGTTTTTACAATTGAAAACACGCTCTCAGAAGATGT
TGCAGATTACTACTGTTTGCAAAGTGATAACATGCCTCTCACGTTCGGAGGGGGGACCAAGCTGGAAATAAAAC (SEQ ID
NO: 144)

## Claims

1. An antibody or fragment thereof specifically binding to tyrosinase-related protein 2 (TRP2), **characterized in** comprising
- as heavy chain variable domain a CDR3 region, said CDR3 region has an amino acid sequence being at least 90% identical to one selected from those in SEQ ID NO: 1, 3, 5, 7, 9, 11, 13, or 15, and/or
- as light chain variable domain a CDR3 region, said CDR3 region has an amino acid sequence being at least 90% identical to one selected from those in SEQ ID NO: 2, 4, 6, 8, 10, 12, 14, or 16.

2. The antibody or fragment of claim 1, **characterized in that**
- the heavy chain variable domain comprises a CDR2 region, said CDR2 region has an amino acid sequence being at least 90% identical to one selected from those in SEQ ID NO: 17, 19, 21, 23, 25, 27, 29, or 31, and/or
- the light chain variable domain comprises a CDR2 region, said CDR2 region has an amino acid sequence being at least 90% identical to one selected from those in SEQ ID NO: 18, 20, 22, 24, 26, 28, 30, or 32.

3. The antibody or fragment of claim 1 or 2, **characterized in that**
- the heavy chain variable domain comprises a CDR1 region, said CDR1 region has an amino acid sequence being at least 90% identical to one selected from those in SEQ ID NO: 33, 35, 37, 39, 41, 43, 45, or 47, and/or
- the light chain variable domain comprises a CDR1 region, said CDR1 region has an amino acid sequence being at least 90% identical to one selected from those in SEQ ID NO: 34, 36, 38, 40, 42, 44, 46, or 48.

4. The antibody or fragment of any of claims 1-3, **characterized in** comprising
- the heavy chain variable domains
- CDR3 region having an amino acid sequence being at least 90% identical to said in SEQ ID NO: 1 and/or
- CDR2 region having an amino acid sequence being at least 90% identical to said in SEQ ID NO: 17 and/or
- CDR1 region having an amino acid sequence being at least 90% identical to said in SEQ ID NO: 33 and/or
- the light chain variable domains
- CDR3 region having an amino acid sequence being at least 90% identical to said in SEQ ID NO: 2 and/or
- CDR2 region having an amino acid sequence being at least 90% identical to said in SEQ ID NO: 18 and/or
- CDR1 region having an amino acid sequence being at least 90% identical to said in SEQ ID NO: 34.

5. The antibody or fragment of any of claims 1-3, **characterized in** comprising
- the heavy chain variable domains
- CDR3 region having an amino acid sequence being at least 90% identical to said in SEQ ID NO: 3 and/or
- CDR2 region having an amino acid sequence being at least 90% identical to said in SEQ ID NO: 19 and/or
- CDR1 region having an amino acid sequence being at least 90% identical to said in SEQ ID NO: 35 and/or
- the light chain variable domains
- CDR3 region having an amino acid sequence being at least 90% identical to said in SEQ ID NO: 4 and/or
- CDR2 region having an amino acid sequence being at least 90% identical to said in SEQ ID NO: 20 and/or
- CDR1 region having an amino acid sequence being at least 90% identical to said in SEQ ID NO: 36.

6. The antibody of fragment thereof or any of the preceding claims comprising
- a heavy chain having an amino acid sequence being at least 90% identical to said in SEQ ID NO:49, and/or
- a light chain having an amino acid sequence being at least 90% identical to said in SEQ ID 50.

7. The antibody of fragment thereof or any of the preceding claims comprising
- a heavy chain having an amino acid sequence being at least 90% identical to said in SEQ ID NO:51, and/or
- a light chain having an amino acid sequence being at least 90% identical to said in SEQ ID 52.

8. The antibody or fragment thereof of any of the preceding claims, **characterized in that** it specifically binds to mouse and/or human TRP2.

9. An antibody or fragment thereof configured for a specific binding to tyrosinase-related protein 2 (TRP2) for use in the prophylaxis and/or treatment of cancer, preferably melanoma.

10. The antibody or fragment thereof of claim 9, which is the antibody or fragment thereof of any of claims 1-8.

11. A pharmaceutical composition **characterized in** comprising an antibody or fragment thereof according to any of claims 1-10.

12. A nucleic acid encoding an antibody or fragment thereof according to any of claims 1-10.

13. A vector comprising the nucleic acid of claim 11 and, optionally, regulatory elements necessary for an expression in a prokaryotic and/or eukaryotic cell.

14. A prokaryotic or eukaryotic host cell comprising the vector of claim 13.

15. A method for the prophylaxis and/or treatment of cancer, preferably melanoma, in a living being, comprising the administration of a prophylactically and/or therapeutically effective amount of the antibody or fragment thereof according to any of claims 1-10, and/or the pharmaceutical composition of claim 11.
